# EUROPEAN PATENT APPLICATION

(11) **EP 4 393 510 A1**
(43) Date of publication of application: **03.07.2024**
(21) Application number: 22876531.9
(22) Date of filing: 30.09.2022
(51) Int. Cl.: A61K 47/02, A61K 9/08, A61K 31/79, A61K 33/30, A61K 47/32, A61P 27/02, A61P 27/04, A61P 31/04, A61P 43/00

(54) **OPHTHALMOLOGICAL COMPOSITION**

(30) Priority: 30.09.2021 JP 2021161918
(71) Applicant: Rohto Pharmaceutical Co., Ltd., Osaka-shi, Osaka 544-8666 (JP)
(72) Inventor: MORIKAWA Takuto, Osaka-shi, Osaka 544-8666 (JP); MATSUMOTO Sachiko, Osaka-shi, Osaka 544-8666 (JP); MATSUKI Rie, Osaka-shi, Osaka 544-8666 (JP)
(74) Representative: Mewburn Ellis LLP
(86) International application number: PCT/JP2022/036673
(87) International publication number: WO 2023/054669

(57) **Abstract**

The present invention relates to an ophthalmological composition containing zinc chloride (A) and a polyvinyl-based polymer compound (B).

## Description

### Technical Field

The present invention relates to an ophthalmological composition.

### Background Art

Usually, multi-dose eye drops contain a preservative having an action of inhibiting microorganism growth in case the eye drops are contaminated with microorganisms such as bacteria or fungi. Incidentally, preservatives also contain a component having cytotoxicity and have been reported to cause corneal epithelial disorder when eye drops containing a preservative are used frequently or for long period of time. Therefore, there is a need of a preformulation study of an eye drop that inhibits microorganism growth with no use of preservatives and has a reduced risk of corneal epithelial disorder.

As a preformulation study of such a preservative-free eye drop, a technique by which a zinc salt such as zinc chloride is incorporated into an ophthalmological composition has been reported (for example, Patent Literature 1).

### Citation List

### Patent Literature

[Patent Literature 1] Japanese Unexamined Patent Publication No. 2010-504358

### Summary of Invention

### Technical Problem

However, for preservative-free eye drops containing a zinc salt, there is still room for additional improvement in preservation effect (preservative power). An objective of the present invention is to provide a preservative-free ophthalmological composition having an excellent preservation effect.

### Solution to Problem

The present inventors found that the preservation effect is significantly enhanced in an ophthalmological composition containing zinc chloride and a polyvinyl-based polymer compound such as polyvinyl pyrrolidone at the same time. The present invention is based on this finding and provides each of the following inventions.
[1] An ophthalmological composition containing zinc chloride (A) and a polyvinyl-based polymer compound (B).
[2] The ophthalmological composition according to [1], in which the polyvinyl-based polymer compound (B) is polyvinyl pyrrolidone.
[3] The ophthalmological composition according to [2], in which a content of the polyvinyl pyrrolidone is 0.001 to 5 w/v% based on a total amount of the ophthalmological composition.
[4] The ophthalmological composition according to [2] or [3], in which the polyvinyl pyrrolidone has a viscosity characteristic value of 10 to 150.
[5] The ophthalmological composition according to any one of [1] to [4], further containing at least one selected from the group consisting of mucopolysaccharides and a cellulose-based polymer compound (C).
[6] The ophthalmological composition according to any one of [1] to [5], not containing benzalkonium chloride.
[7] The ophthalmological composition according to any one of [1] to [6], in which a pH is 5.0 to 8.0.
[8] The ophthalmological composition according to any one of [1] to [7] that is for suppressing eye dryness or dry eye.
[9] The ophthalmological composition according to any one of [1] to [8] that is for a contact lens.
[10] The ophthalmological composition according to any one of [1] to [8] that is for a soft contact lens.

### Advantageous Effects of Invention

According to the present invention, it is possible to provide a preservative-free ophthalmological composition having an excellent preservation effect.

### Description of Embodiments

Hereinafter, an embodiment of the present invention will be described in detail. However, the present invention is not limited to the following embodiment.

In the present specification, unless particularly otherwise described, the unit of contents "%" means "w/v%" and is the same meaning as "g/100 mL."

### [1. Ophthalmological composition]

An ophthalmological composition according to the present embodiment contains zinc chloride "also simply referred to as "component (A)"" and a polyvinyl-based polymer compound "also simply referred to as "component (B)"."

Zinc chloride is not particularly limited as long as the zinc chloride is pharmaceutically, pharmacologically (pharmacologically) or physiologically acceptable.

The content of the component (A) in the ophthalmological composition according to the present embodiment is not particularly limited and is set as appropriate depending on the kinds and contents of other components incorporated, the use, formulation and the like of the ophthalmological composition. The total content of the component (A) may be 0.000001 w/v% or more, 0.00005 w/v% or more or 0.0001 w/v% or more, may be 0.05 w/v% or less, 0.025 w/v% or less or 0.015 w/v% or less and may be 0.000001 to 0.05 w/v%, 0.00005 to 0.025 w/v% or 0.0001 to 0.015 w/v% based on the total amount of the ophthalmological composition from the viewpoint of more significantly exhibiting the effect of the present invention.

The polyvinyl-based polymer compound is not particularly limited as long as the polyvinyl-based polymer compound is pharmaceutically, pharmacologically (pharmacologically) or physiologically acceptable.

Examples of the polyvinyl-based polymer compound include carboxyvinyl polymers, polyvinyl alcohols (fully or partially saponified products), polyvinyl pyrrolidone and salts thereof. The molecular weight of a polyvinyl-based polymer that can be used in the present invention is not particularly limited, and a polyvinyl-based polymer having a weight-average molecular weight of, for example, approximately 5000 to 5,000,000, preferably 10,000 to 3,000,000 and more preferably 10,000 to 1,000,000 can be used. The viscosity characteristic value (K value) of polyvinyl pyrrolidone is not particularly limited, and polyvinyl pyrrolidone having a viscosity characteristic value of preferably 10 to 150 and more preferably 20 to 100 can be used. Particularly, from the viewpoint of further enhancing the effect of the present invention, polyvinyl pyrrolidone is preferable, polyvinyl pyrrolidone K25, polyvinyl pyrrolidone K30 and polyvinyl pyrrolidone K90 are more preferable, and polyvinyl pyrrolidone K30 and polyvinyl pyrrolidone K90 are still more preferable.

As the polyvinyl-based polymer compound, a commercially available product can also be used. One polyvinyl-based polymer compound may be used singly or two or more polyvinyl-based polymer compounds may be used in combination.

The content of the component (B) in the ophthalmological composition according to the present embodiment is not particularly limited and is set as appropriate depending on the kind of the component (B), the kinds and contents of other components incorporated, the use, formulation and the like of the ophthalmological composition. The content of the component (B) may be 0.0001 w/v% or more, 0.0005 w/v% or more, 0.001 w/v% or more or 0.005 w/v% or more, may be 10 w/v% or less, 5 w/v% or less, 3 w/v% or less or 1 w/v% or less and may be 0.0001 to 10 w/v%, 0.0005 to 5 w/v%, 0.001 to 3 w/v% or 0.005 to 1 w/v% based on the total amount of the ophthalmological composition from the viewpoint of more significantly exhibiting the effect of the present invention.

In the case of using polyvinyl pyrrolidone as the component (B), the content of the polyvinyl pyrrolidone in the ophthalmological composition according to the present embodiment is not particularly limited and is set as appropriate depending on the kind of the polyvinyl pyrrolidone, the kinds and contents of other components incorporated, the use, formulation and the like of the ophthalmological composition. The content of the polyvinyl pyrrolidone may be 0.0001 w/v% or more, 0.0005 w/v% or more, 0.001 w/v% or more, 0.005 w/v% or more or 0.01 w/v% or more, may be 10 w/v% or less, 5 w/v% or less, 3 w/v% or less, 1 w/v% or less or 0.5 w/v% or less and may be 0.0001 to 10 w/v%, 0.0005 to 5 w/v%, 0.001 to 3 w/v%, 0.005 to 1 w/v% or 0.01 to 0.5 w/v% based on the total amount of the ophthalmological composition from the viewpoint of more significantly exhibiting the effect of the present invention.

The content ratio of the component (B) to the component (A) in the ophthalmological composition according to the present embodiment is not particularly limited and is set as appropriate depending on the kind of the component (B), the kinds and contents of other components incorporated, the use, formulation and the like of the ophthalmological composition. Regarding the content ratio of the component (B) to the component (A), the total content of the component (B) may be 0.002 parts by mass or more, 0.01 parts by mass or more, 0.06 parts by mass or more or 0.1 parts by mass or more, may be 2000000 parts by mass or less, 100000 parts by mass or less, 10000 parts by mass or less or 1000 parts by mass or less and may be 0.002 to 2000000 parts by mass, 0.01 to 100000 parts by mass, 0.06 to 10000 parts by mass or 0.1 to 1000 parts by mass with respect to 1 part by mass of the total content of the component (A) that is contained in the ophthalmological composition according to the present embodiment from the viewpoint of more significantly enhancing the effect of the present invention.

In the case of using polyvinyl pyrrolidone as the component (B), the content ratio of the polyvinyl pyrrolidone to the component (A) is not particularly limited and is set as appropriate depending on the kind of the polyvinyl pyrrolidone, the kinds and contents of other components incorporated, the use, formulation and the like of the ophthalmological composition. Regarding the content ratio of the polyvinyl pyrrolidone to the component (A), the total content of the polyvinyl pyrrolidone may be 0.002 parts by mass or more, 0.01 parts by mass or more, 0.06 parts by mass or more, 0.1 parts by mass or more or 0.1 parts by mass or more, may be 500 parts by mass or less, 300 parts by mass or less, 200 parts by mass or less, 100 parts by mass or less or less than 100 parts by mass and may be 0.002 to 500 parts by mass, 0.01 to 300 parts by mass, 0.06 to 200 parts by mass, 0.1 to 100 parts by mass or 0.1 parts by mass or more and less than 100 parts by mass with respect to 1 part by mass of the total content of the component (A) that is contained in the ophthalmological composition according to the present embodiment from the viewpoint of more significantly enhancing the effect of the present invention.

The ophthalmological composition according to the present embodiment may further contain at least one selected from the group consisting of mucopolysaccharides and a cellulose-based polymer compound (C) (also referred to as "component (C)"). When the ophthalmological composition further contains the component (C), the effect of the present invention is more significantly exhibited.

The mucopolysaccharides are not particularly limited as long as the mucopolysaccharides are pharmaceutically, pharmacologically (pharmacologically) or physiologically acceptable.

Examples of the mucopolysaccharides include heparin analogs, heparin, heparin sulfate, heparan sulfate, heparinoids, hyaluronic acid and salts thereof and chondroitin sulfate and salts thereof. Examples of such salts include salts with an organic base (basic ammonium salts such as amine salts and arginine and the like), salts with an inorganic base (alkali metal salts such as ammonium salts, sodium salts and potassium salts, alkaline earth metal salts such as calcium salts and magnesium salts, aluminum salts and the like) and the like, and among these, sodium salts, potassium salts and calcium salts are more preferable, and sodium salts are particularly preferable. Among the mucopolysaccharides, hyaluronic acid and salts thereof and chondroitin sulfate and salts thereof are preferable, and chondroitin sulfate and salts thereof are more preferable.

In the case of using mucopolysaccharides as the component (C), the content of the mucopolysaccharides in the ophthalmological composition according to the present embodiment is not particularly limited and is set as appropriate depending on the kind of the mucopolysaccharides, the kinds and contents of other components incorporated, the use, formulation and the like of the ophthalmological composition. The content of the mucopolysaccharides may be 0.00001 w/v% or more, 0.0001 w/v% or more, 0.0005 w/v% or more or 0.001 w/v% or more, may be 10 w/v% or less, 5 w/v% or less, 3 w/v% or less or 1 w/v% or less and may be 0.00001 to 10 w/v%, 0.0001 to 5 w/v%, 0.0005 to 3 w/v% or 0.001 to 1 w/v% based on the total amount of the ophthalmological composition from the viewpoint of more significantly exhibiting the effect of the present invention.

In the case of using the mucopolysaccharides as the component (C), the content ratio of the mucopolysaccharides to the component (A) in the ophthalmological composition according to the present embodiment is not particularly limited and is set as appropriate depending on the kind of the mucopolysaccharides, the kinds and contents of other components incorporated, the use, formulation and the like of the ophthalmological composition. Regarding the content ratio of the mucopolysaccharides to the component (A), for example, the total content of the mucopolysaccharides may be 0.0002 parts by mass or more, 0.001 parts by mass or more, 0.02 parts by mass or more or 0.1 parts by mass or more, may be 2000000 parts by mass or less, 200000 parts by mass or less, 10000 parts by mass or less or 1000 parts by mass or less and may be 0.0002 to 2000000 parts by mass, 0.001 to 200000 parts by mass, 0.02 to 10000 parts by mass or 0.1 to 1000 parts by mass with respect to 1 part by mass of the total content of the component (A) that is contained in the ophthalmological composition according to the present embodiment from the viewpoint of more significantly enhancing the effect of the present invention.

The cellulose-based polymer compound is not particularly limited as long as the cellulose-based polymer compound is pharmaceutically, pharmacologically (pharmacologically) or physiologically acceptable.

Examples of the cellulose-based polymer compound include methylcellulose, ethylcellulose, hydroxyethylcellulose, hydroxymethylcellulose, hydroxypropylcellulose, hydroxypropylmethylcellulose (hypromellose), carboxymethylcellulose, carboxyethylcellulose and salts thereof. Examples of such salts include salts with an organic base (basic ammonium salts such as amine salts and arginine and the like), salts with an inorganic base (alkali metal salts such as ammonium salts, sodium salts and potassium salts, alkaline earth metal salts such as calcium salts and magnesium salts, aluminum salts and the like) and the like, among these, sodium salts, potassium salts and calcium salts are more preferable, and sodium salts are particularly preferable. Among the cellulose-based polymer compounds, hydroxyethylcellulose, hydroxypropylmethylcellulose and salts thereof are preferable, and hydroxypropylmethylcellulose and salts thereof are more preferable.

As the cellulose-based polymer compound, a commercially available product can also be used. One cellulose-based polymer compound may be used singly or two or more cellulose-based polymer compounds may be used in combination.

In the case of using the cellulose-based polymer compound as the component (C), the content of the cellulose-based polymer compound in the ophthalmological composition according to the present embodiment is not particularly limited and is set as appropriate depending on the kind of the cellulose-based polymer compound, the kinds and contents of other components incorporated, the use, formulation and the like of the ophthalmological composition. The content of the cellulose-based polymer compound may be 0.00001 w/v% or more, 0.0001 w/v% or more, 0.0005 w/v% or more, 0.001 w/v% or more, 0.005 w/v% or more or 0.01 w/v% or more, may be 10 w/v% or less, 5 w/v% or less, 3 w/v% or less, 1 w/v% or less, 0.8 w/v% or less or 0.5 w/v% or less and may be 0.00001 to 10 w/v%, 0.0001 to 5 w/v%, 0.0005 to 3 w/v%, 0.001 to 1 w/v%, 0.005 to 0.8 w/v% or 0.01 to 0.5 w/v% based on the total amount of the ophthalmological composition from the viewpoint of more significantly exhibiting the effect of the present invention.

In the case of using the cellulose-based polymer compound as the component (C), the content ratio of the cellulose-based polymer compound to the component (A) in the ophthalmological composition according to the present embodiment is not particularly limited and is set as appropriate depending on the kind of the cellulose-based polymer compound, the kinds and contents of other components incorporated, the use, formulation and the like of the ophthalmological composition. Regarding the content ratio of the cellulose-based polymer compound to the component (A), for example, the total content of the cellulose-based polymer compound may be 0.0002 parts by mass or more, 0.001 parts by mass or more, 0.02 parts by mass or more or 0.1 parts by mass or more, may be 2000000 parts by mass or less, 200000 parts by mass or less, 10000 parts by mass or less or 1000 parts by mass or less and may be 0.0002 to 2000000 parts by mass, 0.001 to 200000 parts by mass, 0.02 to 10000 parts by mass or 0.1 to 1000 parts by mass with respect to 1 part by mass of the total content of the component (A) that is contained in the ophthalmological composition according to the present embodiment from the viewpoint of more significantly enhancing the effect of the present invention.

The ophthalmological composition according to the present embodiment may further contain a buffering agent. When the ophthalmological composition further contains a buffering agent, the effect of the present invention is further exhibited. The buffering agent is not particularly limited as long as the buffering agent is pharmaceutically, pharmacologically (pharmacologically) or physiologically acceptable. Examples of the buffering agent include inorganic buffering agents, which are buffering agents derived from an inorganic acid, and organic buffering agents, which are buffering agents derived from an organic acid or an organic base.

Examples of the inorganic buffering agents include borate buffering agents, phosphate buffering agents, carbonate buffering agents and the like. Examples of the borate buffering agents include boric acid or salts thereof (alkali metal borates, alkaline earth metal borates and the like). Examples of the phosphate buffering agents include phosphoric acid or salts thereof (alkali metal phosphates, alkaline earth metal phosphates and the like). Examples of the carbonate buffering agents include carbonic acid or salts thereof (alkali metal carbonate, alkaline earth metal carbonate and the like). In addition, as the borate buffering agents, the phosphate buffering agents or the carbonate buffering agents, hydrates of borate, phosphate or carbonate may be used. As more specific examples, boric acid or salts thereof (sodium borate, potassium tetraborate, potassium metaborate, ammonium borate, borax and the like) can be exemplified as the borate buffering agents; phosphoric acid or salts thereof (disodium hydrogen phosphate, sodium dihydrogen phosphate, potassium dihydrogen phosphate, trisodium phosphate, tripotassium phosphate, calcium monohydrogen phosphate, calcium dihydrogen phosphate and the like) can be exemplified as the phosphate buffering agents; carbonic acid or salts thereof (sodium bicarbonate, sodium carbonate, ammonium carbonate, potassium carbonate, calcium carbonate, potassium bicarbonate, magnesium carbonate and the like) can be exemplified as the carbonate buffering agents.

Examples of the organic buffering agents include acetate buffering agents, lactate buffering agents, succinate buffering agents, tris buffering agents, AMPD buffering agents and the like. Examples of the acetate buffering agents include acetic acid or salts thereof (alkali metal salts of acetate, alkaline earth metal salts of acetate and the like). Examples of the lactate buffering agents include lactic acid or salts thereof (alkali metal salts of lactate, alkaline earth metal salts of lactate and the like). Examples of the succinate buffering agents include succinic acid or salts thereof (alkali metal salts of succinate and the like). In addition, as the acetate buffering agents, the lactate buffering agents or the succinate buffering agents, hydrates of acetate, lactate or succinate may be used. As more specific examples, acetic acid or salts thereof (ammonium acetate, sodium acetate, potassium acetate, calcium acetate and the like) can be exemplified as the acetate buffering agents; lactic acid or salts thereof (sodium lactate, potassium lactate, calcium lactate and the like) can be exemplified as the lactate buffering agents; succinic acid or salts thereof (monosodium succinate, disodium succinate and the like) can be exemplified as the succinate buffering agents. Examples of the tris buffering agents include trometamol or salts thereof (trometamol hydrochloride and the like). Examples of the AMPD buffering agents include 2-amino-2-methyl-1,3-propanediol or salts thereof.

As the buffering agent, borate buffering agents (for example, a combination of boric acid and borax and the like) are preferable, boric acid and a salt thereof are more preferable, and a combination of boric acid and borax is still more preferable.

As the buffering agent, a commercially available product may also be used. One buffering agent may be used singly or two or more buffering agents may be used in combination.

The content of the buffering agent in the ophthalmological composition according to the present embodiment is not particularly limited and is set as appropriate depending on the kind of the buffering agent, the kinds and contents of other components incorporated, the use, formulation and the like of the ophthalmological composition. The total content of the buffering agent may be 0.0001 w/v% or more, 0.001 w/v% or more or 0.005 w/v% or more, may be 10 w/v% or less, 5 w/v% or less or 3 w/v% or less and may be 0.0001 to 10 w/v%, 0.001 to 5 w/v% or 0.005 to 3 w/v% based on the total amount of the ophthalmological composition from the viewpoint of more significantly exhibiting the effect of the present invention.

The content ratio of the buffering agent to the component (A) in the ophthalmological composition according to the present embodiment is not particularly limited and is set as appropriate depending on the kind of the buffering agent, the kinds and contents of other components incorporated, the use, formulation and the like of the ophthalmological composition. Regarding the content ratio of the buffering agent to the component (A), for example, the total content of the buffering agent may be 0.002 parts by mass or more, 0.02 parts by mass or more or 0.1 parts by mass or more, may be 2000000 parts by mass or less, 50000 parts by mass or less or 2000 parts by mass or less and may be 0.002 to 2000000 parts by mass, 0.02 to 50000 parts by mass or 0.1 to 2000 parts by mass with respect to 1 part by mass of the total content of the component (A) that is contained in the ophthalmological composition according to the present embodiment from the viewpoint of more significantly enhancing the effect of the present invention.

As the buffering agent, it is also possible to use a citrate buffering agent (citric acid or a salt thereof, specifically, citric acid; an alkali metal salt of citrate such as sodium citrate, potassium citrate, sodium dihydrogen citrate or disodium citrate; an alkaline earth metal salt of citrate such as calcium citrate). In this case, the content of the citrate buffering agent in the ophthalmological composition according to the present embodiment may be 0 w/v% or more, 0.0001 w/v% or more or 0.005 w/v% or more, may be 0.1 w/v% or less, 0.08 w/v% or less or 0.05 w/v% or less and may be 0 to 0.1 w/v%, 0.0001 to 0.08 w/v% or 0.005 to 0.05 w/v% based on the total amount of the ophthalmological composition from the viewpoint of more significantly exhibiting the effect of the present invention. The citrate buffering agent may not be contained (0 w/v%).

The ophthalmological composition according to the present embodiment may further contain a surfactant. When the ophthalmological composition further contains a surfactant, the effect of the present invention is further exhibited. The surfactant is not particularly limited as long as the surfactant is pharmaceutically, pharmacologically (pharmacologically) or physiologically acceptable, and the surfactant may be any of a nonionic surfactant, an amphoteric surfactant, an anionic surfactant or a cationic surfactant.

Examples of the nonionic surfactant include POE sorbitan fatty acid esters such as POE (20) sorbitan monolaurate (polysorbate 20), POE (20) sorbitan monopalmitate (polysorbate 40), POE (20) sorbitan monostearate (polysorbate 60), POE (20) sorbitan tristearate (polysorbate 65) and POE (20) sorbitan monooleate (polysorbate 80); POE hydrogenated castor oils such as POE (40) hydrogenated castor oil (polyoxyethylene hydrogenated castor oil 40) and POE (60) hydrogenated castor oil (polyoxyethylene hydrogenated castor oil 60); POE castor oils such as POE (3) hydrogenated castor oil (polyoxyethylene castor oil 3), POE (10) castor oil (polyoxyethylene castor oil 10) and POE (35) castor oil (polyoxyethylene castor oil 35); POE alkyl ethers such as POE (9) lauryl ether; POE-POP alkyl ethers such as POE (20) POP (4) cetyl ether; polyethylene glycol monostearate such as polyoxyl 40 stearate; POE/POP block copolymers such as POE (196) POP (67) glycol (poloxamer 407, PLURONIC F127) and POE (200) POP (70) glycol; and the like. In the above-exemplified compounds, POE indicates polyoxyethylene, POP indicates polyoxypropylene, and numbers in the parentheses indicate the number of moles added, respectively.

Examples of the amphoteric surfactant include alkyldiaminoethylglycine or salts thereof (for example, hydrochloride and the like).

Examples of the anionic surfactant include alkylbenzene sulfonate, alkyl sulfate, polyoxyethylene alkyl sulfate, aliphatic α-sulfomethyl ester, α-olefin sulfonic acid and the like.

Examples of the cationic surfactant include cetylpyridinium chloride, benzalkonium chloride, benzethonium chloride and the like.

Among these surfactants, the nonionic surfactants are preferable, and POE sorbitan fatty acid esters, POE hydrogenated castor oils, POE castor oils, polyethylene glycol monostearate and POE/POP block copolymers are more preferable.

As the surfactant, a commercially available product may also be used. One surfactant may be used singly or two or more surfactants may be used in combination.

The content of the surfactant in the ophthalmological composition according to the present embodiment is not particularly limited and is set as appropriate depending on the kind of the surfactant, the kinds and contents of other components incorporated, the use, formulation and the like of the ophthalmological composition. The total content of the surfactant may be 0.001 w/v% or more, 0.005 w/v% or more or 0.01 w/v% or more, may be 5 w/v% or less, 3 w/v% or less or 1 w/v% or less and may be 0.001 to 5 w/v%, 0.005 to 3 w/v% or 0.01 to 1 w/v% based on the total amount of the ophthalmological composition from the viewpoint of more significantly exhibiting the effect of the present invention.

The content ratio of the surfactant to the component (A) in the ophthalmological composition according to the present embodiment is not particularly limited and is set as appropriate depending on the kind of the surfactant, the kinds and contents of other components incorporated, the use, formulation and the like of the ophthalmological composition. Regarding the content ratio of the surfactant to the component (A), for example, the total content of the surfactant may be 0.002 parts by mass or more, 0.1 parts by mass or more or 1 part by mass or more, may be 60000 parts by mass or less, 10000 parts by mass or less or 1000 parts by mass or less and may be 0.002 to 60000 parts by mass, 0.1 to 10000 parts by mass or 1 to 1000 parts by mass with respect to 1 part by mass of the total content of the component (A) that is contained in the ophthalmological composition according to the present embodiment from the viewpoint of more significantly enhancing the effect of the present invention.

The ophthalmological composition according to the present embodiment may further contain a chelating agent. The chelating agent is not particularly limited as long as the chelating agent is pharmaceutically, pharmacologically (pharmacologically) or physiologically acceptable.

Examples of the chelating agent include ethylenediaminetetraacetic acid (EDTA; edetic acid), ethylenediaminediacetic acid (EDDA), ethylenediaminetriacetic acid, N-(2-hydroxyethyl)ethylenediaminetriacetic acid (HEDTA), diethylenetriaminepentaacetic acid (DTPA) and salts thereof. Examples of such salts include salts with an inorganic base (alkali metal salts such as ammonium salts, sodium salts and potassium salts, alkaline earth metal salts such as calcium salts and magnesium salts, aluminum salts and the like) and the like, among these, sodium salts and potassium salts are more preferable, and sodium salts are particularly preferable. Among the chelating agents, edetic acid and a salt thereof are preferable, sodium edetate, disodium edetate and tetrasodium edetate are more preferable, and disodium edetate is still more preferable.

The content of the chelating agent in the ophthalmological composition according to the present embodiment is not particularly limited and is set as appropriate depending on the kind of the chelating agent, the kinds and contents of other components incorporated, the use, formulation and the like of the ophthalmological composition. The content of the chelating agent may be 0 w/v% or more, may be 0.01 w/v% or less, 0.001 w/v% or less or 0.0001 w/v% or less and may be 0 to 0.01 w/v%, 0 to 0.001 w/v% or 0 to 0.0001 w/v% based on the total amount of the ophthalmological composition from the viewpoint of more significantly exhibiting the effect of the present invention. The chelating agent may not be contained (0 w/v%).

The content ratio of the chelating agent to the component (A) in the ophthalmological composition according to the present embodiment is not particularly limited and is set as appropriate depending on the kind of the chelating agent, the kinds and contents of other components incorporated, the use, formulation and the like of the ophthalmological composition. Regarding the content ratio of the chelating agent to the component (A), for example, the total content of the chelating agent may be 0 parts by mass or more, may be 2000 parts by mass or less, 100 parts by mass or less or 20 parts by mass or less and may be 0 to 2000 parts by mass, 0 to 100 parts by mass or 0 to 20 parts by mass with respect to 1 part by mass of the total content of the component (A) that is contained in the ophthalmological composition according to the present embodiment from the viewpoint of more significantly enhancing the effect of the present invention.

The pH of the ophthalmological composition according to the present embodiment is not particularly limited as long as the pH is within a pharmaceutically, pharmacologically (pharmacologically) or physiologically acceptable range. The pH of the ophthalmological composition according to the present embodiment may be 4.0 or higher, 4.5 or higher, 5.0 or higher or 5.5 or higher, may be 9.5 or lower, 9.0 or lower, 8.0 or lower, 7.5 or lower, 7.0 or lower or lower than 7.0 and may be 4.0 to 9.5, 4.5 to 9.0, 5.0 to 8.0, 5.5 to 7.5 or 5.5 to 7.0. The pH of the ophthalmological composition according to the present embodiment may be 5.0 or higher and lower than 7.0 or 5.5 or higher and lower than 7.0 from the viewpoint of more significantly exhibiting the effect of the present invention.

The ophthalmological composition according to the present embodiment can be adjusted to be an osmotic pressure ratio within a biologically acceptable range as necessary. An appropriate osmotic pressure ratio can be set as appropriate depending on the use, formulation, usage method or the like of the ophthalmological composition and may be 0.4 or more, 0.6 or more or 0.8 or more, may be 5.0 or less, 3.0 or less, 2.2 or less or 2.0 or less and may be 0.4 to 5.0, 0.6 to 3.0, 0.8 to 2.2 or 0.8 to 2.0. The osmotic pressure ratio is defined as the ratio of the osmotic pressure of a specimen to 286 mOsm (the osmotic pressure of a 0.9 w/v% sodium chloride aqueous solution) based on Japanese Pharmacopoeia, 17^{th} Edition, and the osmotic pressure ratio is measured with reference to the osmometry (freezing point depression method) described in Japanese Pharmacopoeia. The standard solution for osmotic pressure ratio measurement (0.9 w/v% sodium chloride aqueous solution) can be prepared by drying sodium chloride (Japanese Pharmacopoeia standard reagent) at 500°C to 650°C for 40 to 50 minutes, then, leaving the sodium chloride to stand in a desiccator (silica gel), accurately weighing 0.900 g of the sodium chloride and dissolving the sodium chloride in purified water to make an accurate volume of 100 mL or a commercially available standard solution for osmotic pressure ratio measurement (0.9 w/v% sodium chloride aqueous solution) can be used.

The viscosity of the ophthalmological composition according to the present embodiment is not particularly limited as long as the viscosity is within a pharmaceutically, pharmacologically (pharmacologically) or physiologically acceptable range. Regarding the viscosity of the ophthalmological composition according to the present embodiment, for example, the viscosity measured at 20°C with a rotational viscometer (TV-20 viscometer, manufactured by Toki Sangyo Co., Ltd., rotor; 1°34' × R24) may be 1 mPa·s or higher or 1.1 mPa·s or higher, may be 10000 mPa·s or lower, 8000 mPa·s or lower, 1000 mPa·s or lower, 100 mPa·s or lower, 20 mPa·s or lower or 17 mPa·s or lower and may be 1 to 10000 mPa·s, 1 to 8000 mPa·s, 1 to 1000 mPa·s, 1 to 100 mPa·s, 1 to 20 mPa·s or 1.1 to 17 mPa·s.

The ophthalmological composition according to the present embodiment may contain an appropriate amount of one or more components selected from various pharmacologically active components and physiologically active components in addition to the above-described components to an extent that the effect of the present invention is not impaired. The components are not particularly limited, and, for example, active components in ophthalmological drugs described in Approval Criteria for Marketing Guidance-Required Over-the-Counter Pharmaceuticals, 2017 (edited by General incorporated association Society for Regulatory Science of Medical Products) can be exemplified. Specific examples of the components that are used in ophthalmological drugs include the following components.

Anti-allergic agents: For example, sodium cromoglycate, tranilast, potassium pemirolast, acitazanolast, amlexanox, ibudilast and the like.

Antihistamines: For example, chlorpheniramine or a salt thereof (for example, chlorpheniramine maleate), diphenhydramine or a salt thereof (for example, diphenhydramine hydrochloride), iproheptine or a salt thereof (for example, iproheptine hydrochloride), levocabastine or a salt thereof (for example, levocabastine hydrochloride), ketotifen or a salt thereof (for example, ketotifen fumarate), pemirolast potassium, olopatadine or a salt thereof (for example, olopatadine hydrochloride) and the like.

Anti-inflammatory agents: For example, methyl salicylate, glycol salicylate, allantoin, tranexamic acid, lysozyme, lysozyme chloride, indomethacin, pranoprofen, ibuprofen, ibuprofen piconol, ketoprofen, felbinac, bendazac, piroxicam, bufexamac, butyl flufenamate, epsilon-aminocaproic acid, berberine chloride, berberine sulfate, sodium azulene sulfonate, glycyrrhizic acid or a salt thereof (for example, dipotassium glycyrrhizinate or monoammonium glycyrrhizinate) and the like.

Steroids: For example, fluticasone propionate, fluticasone furoate, mometasone furoate, beclomethasone propionate, flunisolide and the like.

Decongestants: For example, tetrahydrozoline hydrochloride, tetrahydrozoline nitrate, naphazoline hydrochloride, naphazoline nitrate, epinephrine, epinephrine hydrochloride, ephedrine hydrochloride, phenylephrine hydrochloride, dl-methylephedrine hydrochloride and the like.

Eye muscle regulating agents: For example, cholinesterase inhibitors having an active center similar to acetylcholine, specifically, neostigmine methyl sulfate, tropicamide, helenien, atropine sulfate, pilocarpine hydrochloride and the like.

Vitamins: For example, retinol acetate, retinol palmitate, tocopherol acetate, sodium flavin adenine dinucleotide, cyanocobalamin, pyridoxine hydrochloride, panthenol, calcium pantothenate, ascorbic acid, sodium ascorbate, thiamine hydrochloride and the like.

Amino acids: For example, L-arginine, glutamic acid, glycine, alanine, lysine, γ-aminobutyric acid, γ-aminovaleric acid, trimethylglycine, taurine, aspartic acid, salts thereof and the like.

Astringents: For example, zinc white, zinc lactate, zinc sulfate and the like.

Others: For example, sulfamethoxazole, sulfisoxazole, sulfisomidine, their salts thereof and the like.

The ophthalmological composition according to the present embodiment may contain an appropriate amount of one or more of a variety of additives that are selected as appropriate and jointly used according to a normal method depending on the use and formulation of the ophthalmological composition to an extent that the effect of the present invention is not impaired. As such additives, for example, a variety of additives described in Japanese Pharmaceutical Excipients Directory 2016 (edited by IPEC JAPAN) can be exemplified. Examples of typical components include the following additives.

Carriers: For example, water and aqueous solvents such as moisture ethanol.

Bases: For example, octyldodecanol, titanium oxide, potassium bromide, plastibase and the like.

pH regulators: For example, hydrochloric acid, acetic acid, sodium hydroxide, potassium hydroxide, calcium hydroxide, magnesium hydroxide, triethanolamine, monoethanolamine, diisopropanolamine and the like.

Fragrances or cooling agents: For example, menthol, menthone, camphor, borneol, geraniol, cineol, citronellol, carvone, anethole, eugenol, limonene, linalool, linalyl acetate, thymol, cymene, terpineol, pinene, camphene, isoborneol, fenchene, nerol, myrcene, myrcenol, linalool acetate, lavandulol, eucalyptus oil, bergamot oil, peppermint oil, coolmint oil, spearmint oil, peppermint oil, fennel oil, cinnamon bark oil, rose oil, camphor oil and the like. These may be any of a d-form, an l-form or a dl-form.

Thickeners: For example, starch; chitin and derivatives thereof; chitosan and derivatives thereof; carrageenan; monosaccharides such as glucose and the like.

Stabilizers: For example, sodium formaldehyde sulfoxylate (Rongalit), aluminum monostearate, glyceryl monostearate, cyclodextrin, monoethanolamine, dibutylhydroxytoluene, sodium bisulfite, sodium pyrosulfite and the like.

Preservatives: For example, quaternary ammonium salts of alkyl polyaminoethylglycines (for example, benzalkonium chloride, benzethonium chloride and the like), chlorhexidine gluconate, polydronium chloride, sodium benzoate, ethanol, chlorobutanol, sorbic acid, potassium sorbate, sodium dehydroacetate, methyl paraoxybenzoate, ethyl paraoxybenzoate, propyl paraoxybenzoate, butyl paraoxybenzoate, oxyquinoline sulfate, phenethyl alcohol, benzyl alcohol, biguanide compounds (specifically, polyhexanide hydrochloride (polyhexamethylene biguanide), alexidine and the like), GLOKILL (manufactured by Rhodia, product name) and the like.

Isotonic agents: For example, potassium chloride, calcium chloride, sodium chloride, magnesium chloride, potassium acetate, sodium acetate, sodium bicarbonate, sodium carbonate, sodium thiosulfate, magnesium sulfate, glycerin, propylene glycol, sodium bisulfite, sodium sulfite and the like.

Sugar alcohols: For example, xylitol, sorbitol, mannitol, glycerin and the like. These may be any of a d-form, an l-form or a dl-form.

Oils: For example, vegetable oils such as sesame oil, castor oil, soybean oil and olive oil; animal oils such as squalane; mineral oils such as liquid paraffin and petrolatum and the like.

From the viewpoint of enabling the effect of the present invention to be significantly exhibited, the ophthalmological composition according to the present embodiment preferably does not contain benzalkonium chloride, more preferably does not contain at least one selected from the group consisting of benzalkonium chloride, methyl paraoxybenzoate, ethyl paraoxybenzoate, propyl paraoxybenzoate and butyl paraoxybenzoate and still more preferably does not contain at least one selected from the group consisting of a biguanide compound, alkyl polyaminoethylglycines, chlorhexidine gluconate, sorbate, a quaternary ammonium salt, methyl paraoxybenzoate, ethyl paraoxybenzoate, propyl paraoxybenzoate and butyl paraoxybenzoate. In addition, the ophthalmological composition according to the present embodiment preferably does not contain any preservatives from the viewpoint of enabling the effect of the present invention to be significantly exhibited.

From the viewpoint of enabling the effect of the present invention to be significantly exhibited, the ophthalmological composition according to the present embodiment preferably does not contain at least one selected from the group consisting of methyl salicylate, glycol salicylate, tranexamic acid, lysozyme, indomethacin, ibuprofen, ibuprofen piconol, ketoprofen, felbinac, bendazac, piroxicam, bufexamac, butyl flufenamate and salts thereof and more preferably does not contain at least one selected from the group consisting of allantoin, epsilon-aminocaproic acid, berberine, azulene sulfonic acid, glycyrrhizic acid, pranoprofen, methyl salicylate, glycol salicylate, tranexamic acid, lysozyme, indomethacin, ibuprofen, ibuprofen piconol, ketoprofen, felbinac, bendazac, piroxicam, bufexamac, butyl flufenamic acid and salts thereof. In addition, the ophthalmological composition according to the present embodiment preferably does not contain any anti-inflammatory agents from the viewpoint of enabling the effect of the present invention to be significantly exhibited.

From the viewpoint of enabling the effect of the present invention to be significantly exhibited, the ophthalmological composition according to the present embodiment preferably does not contain at least one selected from the group consisting of epinephrine, ephedrine, phenylephrine, methylephedrine and salts thereof and more preferably does not contain at least one selected from the group consisting of tetrahydrozoline, naphazoline, epinephrine, ephedrine, phenylephrine, methylephedrineand salts thereof. In addition, the ophthalmological composition according to the present embodiment preferably does not contain any decongestants from the viewpoint of enabling the effect of the present invention to be significantly exhibited.

From the viewpoint of enabling the effect of the present invention to be significantly exhibited, the ophthalmological composition according to the present embodiment preferably does not contain at least one selected from the group consisting of tropicamide, hellenien, atropine, pilocarpine and salts thereof and more preferably does not contain at least one selected from the group consisting of neostigmine, tropicamide, helenien, atropine, pilocarpine and salts thereof. In addition, the ophthalmological composition according to the present embodiment preferably does not contain any eye muscle-regulating drugs from the viewpoint of enabling the effect of the present invention to be significantly exhibited.

From the viewpoint of enabling the effect of the present invention to be significantly exhibited, the ophthalmological composition according to the present embodiment preferably does not contain at least one selected from the group consisting of pantothenic acid, thiamine and salts thereof and more preferably does not contain at least one selected from the group consisting of pyridoxine, panthenol, cyanocobalamin, tocopherol acetate, retinol acetate, retinol palmitate, flavin adenine dinucleotide, ascorbic acid, pantothenic acid, thiamine and salts thereof. In addition, the ophthalmological composition according to the present embodiment preferably does not contain any vitamins from the viewpoint of enabling the effect of the present invention to be significantly exhibited.

From the viewpoint of enabling the effect of the present invention to be significantly exhibited, the ophthalmological composition according to the present embodiment preferably does not contain at least one selected from the group consisting of zinc lactate and zinc white, more preferably does not contain at least one selected from the group consisting of zinc sulfate, zinc lactate and zinc white, and still more preferably does not contain at least one selected from the group consisting of zinc compounds other than zinc chloride. In addition, the ophthalmological composition according to the present embodiment preferably does not contain any astringents from the viewpoint of enabling the effect of the present invention to be significantly exhibited.

The ophthalmological composition according to the present embodiment preferably does not contain rebamipide from the viewpoint of enabling the effect of the present invention to be significantly exhibited. In addition, from the viewpoint of enabling the effect of the present invention to be significantly exhibited, the ophthalmological composition according to the present embodiment preferably does not contain VASELINE, alginic acid, salts thereof and sorbitol, more preferably does not contain VASELINE, alginic acid and salts thereof or VASELINE and sorbitol, and still more preferably does not contain VASELINE.

In a case where the ophthalmological composition according to the present embodiment contains water, regarding the content of water, from the viewpoint of more significantly exhibiting the effect of the present invention, the content of water may be 80 w/v% or more, 85 w/v% or more or 90 w/v%, less than 100 w/v%, 99.5 w/v% or less or 99.2 w/v% or less and may be 80 w/v% or more and less than 100 w/v%, 85 w/v% or more and 99.5 w/v% or less or 90 w/v% or more and 99.2 w/v% or less based on the total amount of the ophthalmological composition.

The water that is used in the ophthalmological composition according to the present embodiment needs to be pharmaceutically, pharmacologically (pharmacologically) or physiologically acceptable. Examples of such water include distilled water, regular water, purified water, sterile purified water, water for injection, distilled water for injection and the like. Definitions thereof are based on Japanese Pharmacopoeia, 17^{th} Edition.

The ophthalmological composition according to the present embodiment can be prepared by adding and mixing desired amounts of the component (A) and the component (B) and, as necessary, other components as much as a desired concentration is reached. For example, the ophthalmological composition can be prepared by dissolving or dispersing those components in purified water, adjusting the pH and the osmotic pressure to be predetermined values and performing sterilization by filtration sterilization or the like.

The ophthalmological composition according to the present embodiment may have a variety of formulations depending on the purposes. Examples of the formulations include liquids, gels, semi-solids (ointments and the like) and the like.

The ophthalmological composition according to the present embodiment can be used as, for example, an eye drop (also referred to as an ophthalmic solution or an eye lotion; the eye drop includes an eye drop that can be instilled into the eyes while wearing contact lenses), artificial tears, an eyewash (also referred to as an eyewash solution or an eyewash lotion; the eyewash includes an eyewash that can be instilled into the eyes while wearing contact lenses) or a composition for contact lens [a contact lens fitting solution, a composition for contact lens care (a contact lens disinfectant, a contact lens preservative, a contact lens cleaning agent or a contact lens cleaning preservative), a contact lens packaging liquid or the like]. "Contact lens" includes hard contact lenses and soft contact lenses (including both ionic and anionic contact lenses and including both silicone hydrogel contact lenses and non-silicone hydrogel contact lenses). The ophthalmological composition according to the present embodiment is preferably an ophthalmological composition for contact lenses from the viewpoint of more significantly exhibiting the effect of the present invention. In addition, the ophthalmological composition according to the present embodiment is more preferably an ophthalmological composition for soft contact lenses from the viewpoint of improvement in easiness in spread immediately after being instilled into the eyes while wearing soft contact lenses and feeling of wearing (reduction of dryness or the like).

The ophthalmological composition according to the present embodiment is preferably an eye drop (including an eye drop that can be instilled into the eyes while wearing contact lenses) since it is possible to more significantly exhibit the effect of the present invention. In a case where the ophthalmological composition according to the present embodiment is an eye drop, the administration and the dose are not particularly limited as long as the effect is exhibited and a side effect is less caused, and a method in which, in the case of, for example, an adult (aged 15 or more years) or a child aged seven or more years, one to three droplets, one or two droplets or two or three droplets are instilled into the eyes two to four times or five or six times a day can be exemplified.

As confirmed in examples to be described below, the ophthalmological composition according to the present embodiment has an effect of suppressing the drying of contact lenses, an effect of reducing friction, an effect of increasing the viscosity and an effect of improving the survival rate of corneal epithelial cells against dryness and can be thus suitably used to suppress eye dryness or dry eye.

The ophthalmological composition according to the present embodiment is provided in a state of being accommodated in an arbitrary container. The container that accommodates the ophthalmological composition according to the present embodiment is not particularly limited and may be, for example, a glass container or a plastic container. A plastic container is preferable. Examples of the plastic include polyethylene terephthalate (PET), polyarylate, polyethylene naphthalate, polycarbonate, polyethylene, polypropylene, polyimide, copolymers of a monomer composing the above-listed plastic and plastic obtained by mixing two or more thereof. Polyethylene terephthalate is preferable. In addition, the container that accommodates the ophthalmological composition according to the present embodiment may be a transparent container through which the inside of the container can be viewed or an opaque container through which it is difficult to view the inside of the container. A transparent container is preferable. Here, "transparent container" includes both colorless transparent containers and colored transparent containers.

The container that accommodates the ophthalmological composition according to the present embodiment may be equipped with a nozzle. The material of the nozzle is not particularly limited and may be, for example, glass or plastic. A plastic nozzle is preferable. Examples of the plastic include polybutylene terephthalate, polyethylene, polypropylene, polyethylene terephthalate, polyethylene naphthalate, copolymers of a monomer composing the above-listed plastic and plastic obtained by mixing two or more thereof. The material of the nozzle is preferably polypropylene, polyethylene, polyethylene terephthalate, polybutylene terephthalate or polyethylene naphthalate and more preferably polyethylene from the viewpoint of further enhancing the effect of the present invention,

The container that accommodates the ophthalmological composition according to the present embodiment may be a multi-dose type in which the ophthalmological composition is contained as much as being used a plurality of times or a single-dose type in which the ophthalmological composition is contained as much as being used a single time, but is preferably a multi-dose type from the viewpoint of exhibiting the effect of the present invention.

The ophthalmological composition according to the present embodiment is preferably loaded into a container having an inner capacity of 4 to 30 mL, more preferably loaded into a container having an inner capacity of 5 to 20 mL, still more preferably loaded into a container having an inner capacity of 6 to 16 mL and far still more preferably loaded into a container having an inner capacity of 10 to 15 mL. In addition, the ophthalmological composition may be loaded into a container having an inner capacity of 0.1 to 3 mL and may be loaded into a container having an inner capacity of 0.2 to 1 mL.

### [2. Method for enhancing preservation effect of ophthalmological composition]

The eye drop according to the present embodiment contains the component (A) and the component (B), whereby the preservation effect improves. Therefore, as one embodiment of the present invention, a method for enhancing the preservation effect of an ophthalmological composition, in which zinc chloride (A) and a polyvinyl-based polymer compound (B) are incorporated into the ophthalmological composition, is provided.

In the method, the kinds, contents and the like of the component (A) and the component (B), the kinds, contents and the like of other components, the use, formulation and the like of the ophthalmological composition are as described in the [1. Ophthalmological composition] section.

### [Examples]

Hereinafter, the present invention will be specifically described based on test examples, but the present invention is not limited thereto.

### [Test Example 1: Preservation effect test (1)]

Each ophthalmological composition was prepared in a composition shown in Tables 1 and 2 according to a normal method and used as a test solution. The unit of each component in Tables 1 and 2 is "w/v%." Escherichia coli (ATCC8739) was implanted into the surface of a soybean casein digest slant medium and cultured at 33°C for 24 hours. A cultured bacterial cell was aseptically collected with a platinum loop and made to float on an appropriate amount of sterile physiological saline, thereby preparing a bacterium floating liquid containing approximately 1 × 10⁷ CFU/mL of living bacteria. The number of the living bacteria in the floating liquid was measured by separately culturing the bacteria. Next, 10 mL of each of the prepared ophthalmological compositions was loaded into a 15 mL CORNING conical tube (PET). A bacterial liquid of Escherichia coli (suspended with physiological saline) was implanted into each of these ophthalmological compositions so that the number of the living bacteria (final concentration) reached approximately 10⁵ CFU/mL, well stirred and used as a specimen. The specimen containing the bacteria was stored with light shielded at 23°C for five days. After that, the specimen containing the bacteria was adjusted so that the concentration became appropriate for counting, 1 mL of the specimen was implanted onto a 3M^{™} Petrifilm ^{™} rapid aerobic count plate (RAC plate) and cultured at 33°C for two days, and the number of observed colonies was counted to obtain the number of living bacteria. The number of living bacteria immediately after the implantation and the number of living bacteria in the specimen after five days of storage were compared, and the percentage of the number of bacteria reduced was calculated as Log Reduction. The bacteria were cultured at 33°C for two days for counting. The results are shown in Tables 1 and 2.

**[Table 1]**

| Component name | Test solution 1-1 | Test solution 1-2 | Test solution 1-3 | Test solution 1-4 | Test solution 1-5 | Test solution 1-6 | Test solution 1-7 | Test solution 1-8 |
|---|---|---|---|---|---|---|---|---|
| Polyvinyl pyrrolidone (K value = 90) | - | - | 0.2 | 0.2 | - | - | - | 0.2 |
| Polyvinyl pyrrolidone (K value = 25) | - | - | - | - | 0.2 | 0.2 | - | - |
| Zinc chloride | - | 0.003 | - | 0.003 | - | 0.003 | 0.05 | 0.05 |
| Boric acid | 1 | 1 | 1 | 1 | 1 | 1 | 1 | 1 |
| Borax | 0.08 | 0.08 | 0.08 | 0.08 | 0.08 | 0.08 | 0.08 | 0.08 |
| Purified water | Residue | Residue | Residue | Residue | Residue | Residue | Residue | Residue |
| Total amount (mL) | 100 | 100 | 100 | 100 | 100 | 100 | 100 | 100 |
| Percentage of number of bacteria reduced (Log Reduction) | 1.03 | 2.41 | 1.95 | 5.81 | 3.21 | 5.81 | 3.51 | 5.81 |

**[Table 2]**

| Component name | Test solution 1-9 | Test solution 1-10 | Test solution 1-11 | Test solution 1-12 |
|---|---|---|---|---|
| Polyvinyl pyrrolidone (K value = 90) | - | - | 0.2 | 0.2 |
| Polyvinyl pyrrolidone (K value = 25) | - | - | - | - |
| Zinc chloride | - | 0.003 | - | 0.003 |
| Boric acid | 1 | 1 | 1 | 1 |
| Borax | 0.08 | 0.08 | 0.08 | 0.08 |
| Chondroitin sulfate sodium | 0.5 | 0.5 | 0.5 | 0.5 |
| Purified water | Residue | Residue | Residue | Residue |
| Total amount (mL) | 100 | 100 | 100 | 100 |
| Percentage of number of bacteria reduced (Log Reduction) | 0.33 | 0.66 | -0.25 | 2.58 |

It was confirmed that, in the case of using Escherichia coli, which is gram-negative bacteria, as bacteria, compared with the ophthalmological compositions containing any one of zinc chloride or polyvinyl pyrrolidone, the preservation effects significantly improved in the ophthalmological compositions containing both zinc chloride and polyvinyl pyrrolidone. In addition, it is known that, in the case of containing chondroitin sulfate sodium, the preservation effect deteriorates, but it was confirmed that, even in the case of containing chondroitin sulfate sodium, compared with the ophthalmological compositions containing any one of zinc chloride or polyvinyl pyrrolidone, the preservation effects significantly improved in the ophthalmological compositions containing both zinc chloride and polyvinyl pyrrolidone.

### [Test Example 2: Preservation effect test (2)]

Each ophthalmological composition was prepared in a composition shown in Table 3 according to a normal method and used as a test solution. The unit of each component in Table 3 is "w/v%." Staphylococcus aureus (ATCC6538) was implanted into the surface of a soybean casein digest slant medium and cultured at 33°C for 24 hours. A cultured bacterial cell was aseptically collected with a platinum loop and made to float on an appropriate amount of sterile physiological saline, thereby preparing a bacterium floating liquid containing approximately 1 × 10⁷ CFU/mL of living bacteria. The number of the living bacteria in the floating liquid was measured by separately culturing the bacteria. Next, 10 mL of each of the prepared ophthalmological compositions was loaded into a 15 mL CORNING conical tube (PET). A bacterial liquid of Staphylococcus aureus (suspended with physiological saline) was implanted into each of these ophthalmological compositions so that the number of the living bacteria (final concentration) reached approximately 10⁵ CFU/mL, well stirred and used as a specimen. The specimen containing the bacteria was stored with light shielded at 23°C for three days. After that, the specimen containing the bacteria was adjusted so that the concentration became appropriate for counting, 1 mL of the specimen was implanted onto a 3M^{™} Petrifilm ^{™} rapid aerobic count plate (RAC plate) and cultured at 33°C for two days, and the number of observed colonies was counted to obtain the number of living bacteria. The number of living bacteria immediately after the implantation and the number of living bacteria in the specimen after three days of storage were compared, and the percentage of the number of bacteria reduced was calculated as Log Reduction. The bacteria were cultured at 33°C for two days for counting. The results are shown in Table 3.

**[Table 3]**

| Component name | Test solution 2-1 | Test solution 2-2 | Test solution 2-3 | Test solution 2-4 |
|---|---|---|---|---|
| Polyvinyl pyrrolidone (K value = 90) | - | - | 0.2 | 0.2 |
| Zinc chloride | - | 0.003 | - | 0.003 |
| Boric acid | 1 | 1 | 1 | 1 |
| Borax | 0.08 | 0.08 | 0.08 | 0.08 |
| Chondroitin sulfate sodium | 0.5 | 0.5 | 0.5 | 0.5 |
| Purified water | Residue | Residue | Residue | Residue |
| Total amount (mL) | 100 | 100 | 100 | 100 |
| Percentage of number of bacteria reduced (Log Reduction) | 0.36 | 1.63 | -0.09 | 2.13 |

It was confirmed that, even in the case of using Staphylococcus aureus, which is gram-positive bacteria, as bacteria, compared with the ophthalmological compositions containing any one of zinc chloride or polyvinyl pyrrolidone, the preservation effects significantly improved in the ophthalmological compositions containing both zinc chloride and polyvinyl pyrrolidone.

### [Test Example 3: Friction test]

Each ophthalmological composition was prepared in a composition shown in Tables 4 and 5 according to a normal method and used as a test solution. The unit of each component in Tables 4 and 5 is "w/v%." Regarding the prepared test solution, the static friction coefficient was evaluated using a Tribomaster static/dynamic friction measuring instrument (model: TL201Sa (manufactured by Trinity-Lab. Inc.), contact: tactile contact, single mode, speed: 1 mm/sec., vertical load: 20 g, distance: 100 mm). Specifically, artificial leather was installed on the stage side of the measuring instrument, and the static friction coefficient was measured in a state where 100 µL of each test solution had been applied onto the artificial leather. The results are shown in Tables 4 and 5.

**[Table 4]**

| Component name | Test solution 3-1 | Test solution 3-2 | Test solution 3-3 | Test solution 3-4 | Test solution 3-5 | Test solution 3-6 |
|---|---|---|---|---|---|---|
| Polyvinyl pyrrolidone (K value = 90) | - | - | 0.2 | 0.2 | - | - |
| Polyvinyl pyrrolidone (K value = 25) | - | - | - | - | 0.2 | - |
| Polyvinyl pyrrolidone (K value = 30) | - | - | - | - | - | 0.2 |
| Zinc chloride | - | 0.003 | - | 0.003 | 0.003 | 0.003 |
| Boric acid | 1 | 1 | 1 | 1 | 1 | 1 |
| Borax | 0.08 | 0.08 | 0.08 | 0.08 | 0.08 | 0.08 |
| Purified water | Residue | Residue | Residue | Residue | Residue | Residue |
| Total amount (mL) | 100 | 100 | 100 | 100 | 100 | 100 |
| Static friction coefficient (Fs) | 1.01 | 1.11 | 1.31 | 1.07 | 1.06 | 0.92 |

**[Table 5]**

| Component name | Test solution 3-7 | Test solution 3-8 | Test solution 3-9 | Test solution 3-10 | Test solution 3-11 | Test solution 3-12 |
|---|---|---|---|---|---|---|
| Polyvinyl pyrrolidone (K value = 90) | 0.2 | 0.2 | 0.2 | 0.2 | 0.2 | 0.2 |
| Zinc chloride | - | 0.003 | - | 0.003 | - | 0.003 |
| Boric acid | 1 | 1 | 1 | 1 | 1 | 1 |
| Borax | 0.08 | 0.08 | 0.08 | 0.08 | 0.08 | 0.08 |
| Chondroitin sulfate sodium | 0.5 | 0.5 | - | - | - | - |
| Sodium hyaluronate | - | - | 0.05 | 0.05 | - | - |
| Hydroxypropylmethylcellulose | - | - | - | - | 0.2 | 0.2 |
| Purified water | Residue | Residue | Residue | Residue | Residue | Residue |
| Total amount (mL) | 100 | 100 | 100 | 100 | 100 | 100 |
| Static friction coefficient (Fs) | 1.81 | 1.51 | 1.53 | 0.79 | 1.58 | 1.42 |

It was confirmed that, compared with the ophthalmological compositions containing polyvinyl pyrrolidone, the static friction coefficients significantly decreased in the ophthalmological compositions containing both zinc chloride and polyvinyl pyrrolidone. In addition, the same tendency was confirmed in the ophthalmological compositions further containing chondroitin sulfate sodium, sodium hyaluronate or hydroxypropylmethylcellulose. In the ophthalmological compositions containing both zinc chloride and polyvinyl pyrrolidone, it is possible to reduce blinking difficulty in case of eye dryness or dry eye or discomfort during blinking.

### [Test Example 4: Contact lens drying test]

Each ophthalmological composition was prepared in a composition shown in Table 6 according to a normal method and used as a test solution. The unit of each component in Table 6 is "w/v%." Five grams of agar was sufficiently dissolved in water heated to 90°C to 100°C under stirring and then cooled to 50°C to 60°C under stirring. The cooled agar liquid was poured into an eye ball mold (a mold created based on the form of an average Japanese eye ball: the size φ of the eye was 24 mm: the base curve was 8.65 mm) and solidified by being cooled at 4°C for three hours or longer. The sufficiently solidified agar gel was removed from the mold, and an eye ball model was fabricated. 4 mL of phosphate buffered saline (sodium chloride: 0.60%, disodium hydrogen phosphate (dodecahydrate): 0.60%, sodium dihydrogen phosphate (dihydrate): 0.05%, pH: 7.4 ± 0.1) was dispensed to each of 12 well plates (BDFalcon, No. 35-3043). Two contact lenses (2-WEEK ACUVUE (Johnson & Johnson K.K)) were immersed in each well and left to stand at room temperature for four hours or longer. Furthermore, 4 mL of each test solution shown in Table 6 below was dispensed to each of 12 separate well plates in the same manner, the previously immersed contact lenses were lightly wiped off with lint-free paper, two of them were immersed in each well and left to stand at room temperature for four hours. Moisture on the immersed contact lenses was lightly wiped off with lint-free paper, and the contact lenses were left to stand at the tip (corneal part) of the fabricated eye ball model. The time elapsing from when the lens had been left to stand was measured, and the appearance was observed according to the following observation criteria. The results are shown in Table 6.

### (Observation criteria)

A time taken for any of the following phenomena to be caused in the contact lens is recorded as "time taken for contact lens to be dried", and the test is ended.
- The contact lens sticks to the eye ball model.
- The periphery of the contact lens is deformed or lifted up.

**[Table 6]**

| Component name | Test solution 4-1 | Test solution 4-2 | Test solution 4-3 | Test solution 4-4 |
|---|---|---|---|---|
| Polyvinyl pyrrolidone (K value = 90) | - | - | 0.2 | 0.2 |
| Zinc chloride | - | 0.003 | - | 0.003 |
| Boric acid | 1 | 1 | 1 | 1 |
| Borax | 0.08 | 0.08 | 0.08 | 0.08 |
| Hydrochloric acid | Appropriate amount | Appropriate amount | Appropriate amount | Appropriate amount |
| Sodium hydroxide | Appropriate amount | Appropriate amount | Appropriate amount | Appropriate amount |
| Purified water | Residue | Residue | Residue | Residue |
| Total amount (mL) | 100 | 100 | 100 | 100 |
| pH | 7 | 7 | 7 | 7 |
| Osmotic pressure (mOsm) | 170 | 170 | 170 | 170 |
| Time taken to be dried | 5 minutes | 5 minutes | 2 hours | 2 hr. 30 min. or longer |

It was confirmed that, compared with the ophthalmological composition containing any one of zinc chloride or polyvinyl pyrrolidone, the times taken for the contact lens to be dried significantly extend in the ophthalmological compositions containing both zinc chloride and polyvinyl pyrrolidone.

When Test solution 4-4 was instilled into the eyes wearing contact lenses, spread immediately after the instillation was favorable, and eye dryness were reduced after five minutes from the instillation compared with Test solution 4-3.

### [Test Example 5: Rheology test]

Each ophthalmological composition was prepared in a composition shown in Tables 7 and 8 according to a normal method and used as a test solution. The unit of each component in Tables 7 and 8 is "w/v%." Regarding the prepared test solution, the viscosities were measured for one minute using a rotational viscometer (RE550 type, manufactured by Toki Sangyo Co., Ltd., rotor; 1°34' × R24) at 20°C and a rotation speed of 50 rpm for Test solutions 5-1 to 5-8 and 5-13 to 5-16 and at 20°C and a rotation speed of 20 rpm for Test solutions 5-9 to 5-12. The results are shown in Tables 7 and 8.

**[Table 7]**

| Component name | Test solution 5-1 | Test solution 5-2 | Test solution 5-3 | Test solution 5-4 |
|---|---|---|---|---|
| Polyvinyl pyrrolidone (K value = 90) | - | - | 0.2 | 0.2 |
| Zinc chloride | - | 0.003 | - | 0.003 |
| Boric acid | 1 | 1 | 1 | 1 |
| Borax | 0.08 | 0.08 | 0.08 | 0.08 |
| Purified water | Residue | Residue | Residue | Residue |
| Total amount (mL) | 100 | 100 | 100 | 100 |
| Viscosity (mPa·s) | 1.23 | 1.19 | 1.62 | 1.84 |

**[Table 8]**

| Component name | Test solution 5-5 | Test solution 5-6 | Test solution 5-7 | Test solution 5-8 | Test solution 5-9 | Test solution 5-10 | Test solution 5-11 | Test solution 5-12 | Test solution 5-13 | Test solution 5-14 | Test solution 5-15 | Test solution 5-16 |
|---|---|---|---|---|---|---|---|---|---|---|---|---|
| Polyvinyl pyrrolidone (K value = 90) | - | - | 0.2 | 0.2 | - | - | 0.2 | 0.2 | - | - | 0.2 | 0.2 |
| Zinc chloride | - | 0.003 | - | 0.003 | - | 0.003 | - | 0.003 | - | 0.003 | - | 0.003 |
| Boric acid | 1 | 1 | 1 | 1 | 1 | 1 | 1 | 1 | 1 | 1 | 1 | 1 |
| Borax | 0.08 | 0.08 | 0.08 | 0.08 | 0.08 | 0.08 | 0.08 | 0.08 | 0.08 | 0.08 | 0.08 | 0.08 |
| Chondroitin sulfate sodium | 0.5 | 0.5 | 0.5 | 0.5 | - | - | - | - | - | - | - | - |
| Sodium hyaluronate | - | - | - | - | 0.05 | 0.05 | 0.05 | 0.05 | - | - | - | - |
| Hydroxyprop ylmethylcellul ose+ | - | - | - | - | - | - | - | - | 0.2 | 0.2 | 0.2 | 0.2 |
| Purified water | Residue | Residue | Residue | Residue | Residue | Residue | Residue | Residue | Residue | Residue | Residue | Residue |
| Total amount (mL) | 100 | 100 | 100 | 100 | 100 | 100 | 100 | 100 | 100 | 100 | 100 | 100 |
| Viscosity (mPa·s) | 1.69 | 1.72 | 2.29 | 2.67 | 6.66 | 8.9 | 14.2 | 15.93 | 2.4 | 2.91 | 3.51 | 3.89 |

It was confirmed that, compared with the ophthalmological composition containing any one of zinc chloride or polyvinyl pyrrolidone, the viscosities increased in the ophthalmological compositions containing both zinc chloride and polyvinyl pyrrolidone.

In addition, the same tendency was confirmed in the ophthalmological compositions further containing chondroitin sulfate sodium, sodium hyaluronate or hydroxypropylmethylcellulose. Retention in the eyes can be improved by increasing the viscosity of the ophthalmological composition.

### [Test Example 6: Cell drying test]

Each ophthalmological composition was prepared in a composition shown in Table 9 according to a normal method and used as a test solution. The unit of each component in Table 9 is "w/v%." Human corneal epithelial cell stain HCE-T cells were implanted into 96 well plates (Corning Incorporated) and cultured in a CO₂ incubator set to 37°C and a CO₂ concentration of 5%. The cells were cultured up to a confluent state under conditions of 37°C, 5% CO₂ and a humidity of 90%. As a growth medium, DMEM/F12 (Thermo Fisher Scientific) to which FCS (DS Pharma Biomedical Co., Ltd.), DMSO (Wako Pure Chemical Industries, Ltd.), recombinant human EGF (R&D Systems, Inc.) and insulin solution human (Sigma-Aldrich) were added until the concentrations reached 5%, 0.5%, 10 ng/mL and 5 µg/mL, respectively, was used. After two to four days elapsed from the beginning of the culture and the cells were in a confluent state, the growth medium was removed from each well by suction, 50 µL of each ophthalmological composition was added to the well, and the cells were incubated for 15 minutes under conditions of 37°C and 5% CO₂. Each ophthalmological composition was removed from each well by suction and then left to stand in a clean bench for 20 minutes to impart drought stress, and the number of living cells was then measured. The number of living cells was measured by adding a cell count reagent Cell Counting Kit-8 (Dojindo Laboratories) to each well in an amount of 10 µL with respect to 100 µL of the medium, performing culture for two to three hours under conditions of 37°C, 5% CO₂ and a humidity of 90% and then measuring the absorbance at 450 nm using an absorbance meter (Molecular Devices, LLC.). The result calculated as a ratio to the absorbance at Test solution 6-1 regarding each ophthalmological composition is shown in Table 9. When the number of living cells is large, the absorbance becomes large.

**[Table 9]**

| Component name | Test solution 6-1 | Test solution 6-2 | Test solution 6-3 | Test solution 6-4 | Test solution 6-5 | Test solution 6-6 |
|---|---|---|---|---|---|---|
| Polyvinyl pyrrolidone (K value = 90) | - | - | 0.2 | 0.2 | 0.2 | 0.2 |
| Zinc chloride | - | 0.003 | - | 0.003 | 0.003 | 0.003 |
| Boric acid | 1 | 1 | 1 | 1 | 1 | 1 |
| Borax | 0.08 | 0.08 | 0.08 | 0.08 | 0.08 | 0.08 |
| Chondroitin sulfate sodium | - | - | - | - | 0.5 | - |
| Sodium hyaluronate | - | - | - | - | - | 0.05 |
| Purified water | Residue | Residue | Residue | Residue | Residue | Residue |
| Total amount (mL) | 100 | 100 | 100 | 100 | 100 | 100 |
| Ratio to Test solution 6-1 | 1.00 | 1.14 | 1.35 | 1.46 | 1.74 | 1.68 |

It was confirmed that, compared with the ophthalmological composition containing any one of zinc chloride or polyvinyl pyrrolidone, the survival rates of corneal epithelial cells against dryness improve in the ophthalmological compositions containing both zinc chloride and polyvinyl pyrrolidone. In addition, it was confirmed that the survival rates of corneal epithelial cells against dryness further improve in the ophthalmological compositions further containing chondroitin sulfate sodium or sodium hyaluronate in addition to zinc chloride and polyvinyl pyrrolidone.

### [Formulation examples]

Eye drops are prepared in formulations shown in Tables 10 to 12 below by a normal method. The unit of the amount of each component in Tables 10 to 12 below is "w/v%" except for components the units of which are clearly shown in the tables.

**[Table 10]**

| Component name | Formulation Example 1 | Formulation Example 2 | Formulation Example 3 | Formulation Example 4 | Formulation Example 5 | Formulation Example 6 | Formulation Example 7 | Formulation Example 8 | Formulation Example 9 | Formulation Example 10 |
|---|---|---|---|---|---|---|---|---|---|---|
| Zinc chloride | 0.001 | 0.003 | 0.0003 | 0.005 | 0.003 | 0.001 | 0.003 | 0.0003 | 0.005 | 0.003 |
| Polyvinyl pyrrolidone (K90) | 0.1 | 0.1 | 0.1 | 0.1 | 0.1 | 1 | 1 | 1 | 1 | 1 |
| Polyvinyl pyrrolidone (K30) | - | - | - | - | - | - | - | - | - | - |
| Polyvinyl pyrrolidone (K25) | - | - | - | - | - | - | - | - | - | - |
| Carboxyvinyl polymer | - | - | - | - | - | - | - | - | - | - |
| Polyvinyl alcohol | - | - | - | - | - | - | - | - | - | - |
| Chondroitin sulfate sodium | 1 | - | - | - | - | - | - | 1 | - | - |
| Sodium hyaluronate | - | 0.05 | - | 0.01 | 0.01 | 0.01 | 0.01 | - | 0.05 | - |
| Hydroxypropylmethyl cellulose | - | - | 0.5 | - | 0.1 | - | 0.1 | - | - | 0.5 |
| Hydroxyethylcellulose | - | - | - | 1 | - | 1 | - | - | - | - |
| Neostigmine methylsulfate | - | 0.005 | - | - | - | - | - | - | 0.005 | - |
| Chlorpheniramine maleate | 0.03 | - | - | - | - | 0.03 | - | - | - | 0.03 |
| Dipotassium glycyrrhizate | - | - | - | 0.5 | - | 0.5 | - | - | - | 0.5 |
| D-α-tocopherol acetate | - | - | - | - | - | 0.2 | - | - | - | - |
| Zinc sulfate | 0.1 | - | - | - | - | - | - | 0.3 | - | - |
| Pyridoxine hydrochloride | - | - | - | - | - | 0.1 | - | 0.1 | - | - |
| Epsilon-aminocaproic acid | - | - | - | - | - | 1 | - | - | - | - |
| Taurine | - | - | 1 | - | - | 1 | - | - | - | - |
| Potassium 1-asparaginases | - | - | - | - | 1 | - | - | - | 0.5 | - |
| Calcium chloride | 0.01 | - | - | - | 0.1 | - | - | 0.01 | 0.2 | - |
| Potassium chloride | 0.01 | 0.01 | 0.05 | 0.01 | - | 0.05 | 0.01 | 0.01 | - | 0.01 |
| Sodium chloride | 0.5 | 1 | 0.1 | 1 | 1 | 0.1 | 1 | 0.5 | 1 | 1 |
| Sodium hydrogen carbonate | - | - | - | 0.01 | - | - | 0.01 | - | - | - |
| Boric acid | 1 | 1 | 1 | 1 | 1 | 1 | 1 | 1 | - | 1 |
| Borax | 0.1 | 0.1 | 0.1 | 0.1 | 0.1 | 0.1 | 0.1 | 0.1 | - | 0.1 |
| Sodium dihydrogen phosphate | - | - | - | - | - | - | - | - | 0.1 | - |
| Potassium dihydrogen phosphate | - | - | - | - | - | - | - | - | 0.1 | - |
| Sodium hydrogen phosphate hydrate | - | - | - | - | - | - | - | - | 1 | - |
| Sodium citrate | 0.02 | 0.01 | - | 0.02 | 1 | 0.01 | - | 0.02 | 1 | 1 |
| Anhydrous citric acid | 0.001 | - | - | 0.02 | - | - | - | 0.02 | 0.001 | - |
| Sodium edetate | 0.01 | 0.1 | 0.1 | - | 0.001 | 0.1 | 0.1 | - | 0.01 | 0.001 |
| Polysorbate 80 | 0.2 | 0.1 | - | 0.5 | 0.1 | - | 0.1 | - | 0.1 | 0.05 |
| Polyoxyethylene hydrogenated castor oil 60 | - | 0.1 | 0.1 | - | 0.1 | 0.1 | 0.1 | 0.5 | 0.1 | - |
| Polyoxyethylene castor oil | - | - | 0.01 | - | - | 0.05 | - | 0.05 | - | - |
| Polyoxyethylene (196) polyoxypropylene (67) glycol | - | - | - | 0.1 | 0.05 | 0.01 | - | - | 0.05 | - |
| 1-Menthol | 0.01 | 0.03 | 0.001 | 0.01 | - | - | 0.001 | 0.01 | 0.01 | - |
| Camphor | - | 0.01 | - | - | - | 0.01 | - | - | - | - |
| Bergamot oil | - | - | - | 0.001 | 0.001 | - | - | 0.001 | - | 0.001 |
| Geraniol | - | - | - | - | - | 0.005 | - | 0.001 | - | - |
| Peppermint oil | - | - | - | 0.001 | 0.001 | - | - | 0.001 | - | 0.001 |
| Sesame oil | - | - | 0.1 | - | 0.001 | - | 0.1 | - | - | 0.002 |
| Polyhexanide hydrochloride | 0.0001 | - | 0.001 | - | - | - | - | - | - | - |
| Trometamol | - | - | - | - | - | - | 0.05 | - | - | - |
| Hydrochloric acid | Appropriate amount | Appropriate amount | Appropriate amount | Appropriate amount | Appropriate amount | Appropriate amount | Appropriate amount | Appropriate amount | Appropriate amount | Appropriate amount |
| Sodium hydroxide | Appropriate amount | Appropriate amount | Appropriate amount | Appropriate amount | Appropriate amount | Appropriate amount | Appropriate amount | Appropriate amount | Appropriate amount | Appropriate amount |
| Purified water | Residue | Residue | Residue | Residue | Residue | Residue | Residue | Residue | Residue | Residue |
| Total amount (mL) | 100 mL | 100 mL | 100 mL | 100 mL | 100 mL | 100 mL | 100 mL | 100 mL | 100 mL | 100 mL |
| pH | 5.5 | 6 | 7 | 5.5 | 6 | 7 | 5.5 | 6 | 7 | 5.5 |

**[Table 11]**

| Component name | Formulation Example 11 | Formulation Example 12 | Formulation Example 13 | Formulation Example 14 | Formulation Example 15 | Formulation Example 16 | Formulation Example 17 | Formulation Example 18 | Formulation Example 19 | Formulation Example 20 |
|---|---|---|---|---|---|---|---|---|---|---|
| Zinc chloride | 0.001 | 0.003 | 0.0003 | 0.005 | 0.003 | 0.001 | 0.003 | 0.0003 | 0.005 | 0.003 |
| Polyvinyl pyrrolidone (K90) | 3 | 3 | 3 | 3 | 3 | - | - | - | - | - |
| Polyvinyl pyrrolidone (K30) | - | - | - | - | - | 0.2 | - | - | - | - |
| Polyvinyl pyrrolidone (K25) | - | - | - | - | - | - | 0.2 | - | - | - |
| Carboxyvinyl polymer | - | - | - | - | - | - | - | 0.1 | - | - |
| Polyvinyl alcohol | - | - | - | - | - | - | - | - | 1 | - |
| Chondroitin sulfate sodium | - | - | 1 | - | - | 1 | - | - | - | - |
| Sodium hyaluronate | 0.05 | - | - | 0.01 | 0.01 | - | 0.01 | - | 0.05 | 0.01 |
| Hydroxyprop ylmethylcellul ose | - | 0.5 | - | - | 0.1 | - | - | 0.5 | - | 0.1 |
| Hydroxyethyl cellulose | - | - | - | 1 | - | - | 1 | - | - | - |
| Neostigmine methylsulfate | 0.005 | - | - | - | - | 0.005 | - | - | 0.005 | - |
| Chlorphenira mine maleate | - | - | - | 0.03 | - | - | 0.03 | - | - | - |
| Dipotassium glycyrrhizate | - | 0.5 | - | - | - | - | - | 0.5 | - | 0.5 |
| D-α-tocopherol acetate | - | - | - | - | - | - | - | - | - | - |
| Zinc sulfate | - | - | - | - | 0.1 | - | 0.3 | - | - | - |
| Pyridoxine hydrochloride | 0.1 | - | - | 0.1 | - | - | 0.1 | - | 0.1 | - |
| Epsilon-aminocaproic acid | - | - | - | - | - | - | - | - | - | - |
| Taurine | - | - | 0.2 | - | - | - | - | 0.2 | - | - |
| Potassium 1-asparaginases | - | - | - | - | - | - | - | - | - | - |
| Calcium chloride | 0.2 | 0.01 | - | - | - | 0.01 | - | - | 0.2 | - |
| Potassium chloride | - | 0.01 | 0.05 | 0.01 | 0.01 | 0.01 | 0.01 | 0.01 | - | 0.05 |
| Sodium chloride | 1 | 0.5 | 0.1 | 1 | 1 | 0.5 | 1 | 1 | 1 | 0.1 |
| Sodium hydrogen carbonate | - | - | - | - | 0.01 | - | 0.1 | - | - | - |
| Boric acid | 1 | 1 | 1 | 1 | 1 | 1 | 1 | - | 1 | 1 |
| Borax | 0.1 | 0.1 | 0.1 | 0.1 | 0.1 | 0.1 | 0.1 | - | 0.1 | 0.1 |
| Sodium dihydrogen phosphate | - | - | - | - | - | - | - | 0.1 | - | - |
| Potassium dihydrogen phosphate | - | - | - | - | - | - | - | 0.1 | - | - |
| Sodium hydrogen phosphate hydrate | - | - | - | - | - | - | - | 1 | - | - |
| Sodium citrate | 1 | 0.01 | 0.02 | 0.02 | - | 0.01 | 0.02 | 1 | - | 1 |
| Anhydrous citric acid | - | - | 0.001 | 0.02 | - | - | 0.02 | 0.001 | - | - |
| Sodium edetate | 0.001 | 0.1 | 0.01 | - | 0.1 | 0.1 | - | 0.01 | 0.1 | 0.001 |
| Polysorbate 80 | - | 0.1 | 0.1 | - | 0.1 | 0.1 | - | 0.1 | 0.1 | - |
| Polyoxyethyl ene hydrogenated castor oil 60 | 0.1 | - | 0.1 | 0.1 | 0.1 | - | 0.1 | - | 0.1 | 0.1 |
| Polyoxyethyl ene castor oil | 0.05 | - | - | 0.05 | - | - | 0.05 | - | - | 0.05 |
| Polyoxyethyl ene (196) polyoxypropy lene (67) glycol | 0.01 | 0.1 | 0.05 | 0.01 | - | - | - | 0.1 | 0.05 | 0.01 |
| l-Menthol | 0.015 | - | 0.001 | 0.01 | 0.01 | 0.015 | 0.001 | 0.01 | 0.01 | - |
| Camphor | 0.01 | - | - | - | - | 0.01 | - | - | - | - |
| Bergamot oil | - | 0.001 | - | - | 0.001 | - | - | 0.001 | - | 0.001 |
| Geraniol | - | - | - | - | - | - | - | - | - | - |
| Peppermint oil | - | 0.001 | - | - | 0.001 | - | - | 0.001 | - | 0.001 |
| Sesame oil | - | 0.005 | 0.1 | - | - | - | 0.1 | - | - | 0.01 |
| Polyhexanide hydrochloride | - | - | - | - | - | 0.001 | - | 0.001 | - | - |
| Trometamol | - | - | - | - | - | - | - | - | - | - |
| Hydrochloric acid | Appropriate amount | Appropriate amount | Appropriate amount | Appropriate amount | Appropriate amount | Appropriate amount | Appropriate amount | Appropriate amount | Appropriate amount | Appropriate amount |
| Sodium hydroxide | Appropriate amount | Appropriate amount | Appropriate amount | Appropriate amount | Appropriate amount | Appropriate amount | Appropriate amount | Appropriate amount | Appropriate amount | Appropriate amount |
| Purified water | Residue | Residue | Residue | Residue | Residue | Residue | Residue | Residue | Residue | Residue |
| Total amount (mL) | 100 mL | 100 mL | 100 mL | 100 mL | 100 mL | 100 mL | 100 mL | 100 mL | 100 mL | 100 mL |
| pH | 6 | 7 | 5.5 | 6 | 7 | 5.5 | 6 | 7 | 5.5 | 6 |

**[Table 12]**

| Component name | Formulation Example 21 | Formulation Example 22 | Formulation Example 23 | Formulation Example 24 | Formulation Example 25 | Formulation Example 26 | Formulation Example 27 |
|---|---|---|---|---|---|---|---|
| Zinc chloride | 0.01 | 0.005 | 0.005 | 0.02 | 0.0001 | 0.001 | 0.001 |
| Polyvinyl pyrrolidone (K90) | 0.1 | - | 0.5 | 1 | 0.3 | 0.5 | 0.5 |
| Polyvinyl pyrrolidone (K30) | - | - | 0.1 | - | - | - | - |
| Polyvinyl pyrrolidone (K25) | - | - | - | 0.05 | 0.2 | 0.05 | 0.05 |
| Carboxyvinyl polymer | - | 0.01 | - | - | - | 0.05 | 0.05 |
| Polyvinyl alcohol | - | 0.5 | - | 0.01 | - | - | - |
| Chondroitin sulfate sodium | 0.5 | 0.5 | 0.1 | 0.5 | - | 3 | 3 |
| Sodium hyaluronate | 0.001 | 0.1 | 0.005 | - | 0.001 | 0.1 | 0.1 |
| Hydroxyethyl cellulose | - | 0.01 | - | - | - | 0.6 | 0.6 |
| Hydroxyprop ylmethylcellul ose | 0.05 | - | - | 0.01 | 0.3 | - | - |
| Potassium 1-asparaginases | - | - | 1 | 1 | 0.5 | 0.5 | 0.5 |
| Magnesium potassium 1-asparaginases | - | 0.5 | - | - | 0.5 | - | - |
| Taurine | - | 0.5 | - | 0.1 | - | 1 | 1 |
| Glucose | - | - | 0.005 | - | - | - | - |
| Potassium chloride | - | - | 0.08 | - | - | 0.01 | 0.05 |
| Sodium chloride | 0.6 | 0.4 | 0.44 | 0.3 | 0.45 | 0.4 | 0.4 |
| 1-Menthol | 0.04 | 0.002 | 0.005 | 0.015 | - | 0.005 | 0.005 |
| d-Camphor | 0.01 | - | 0.003 | 0.003 | - | 0.003 | 0.003 |
| Geraniol | 0.003 | - | - | - | - | - | - |
| Eucalyptus oil | - | 0.002 | - | - | - | - | - |
| Bergamot oil | - | - | - | 0.001 | - | 0.001 | - |
| Peppermint oil | - | 0.001 | - | - | - | 0.001 | - |
| Mint oil | - | 0.002 | - | - | - | - | - |
| Polyoxyethyl ene hydrogenated castor oil | - | - | 0.2 | 0.1 | - | - | - |
| Polyoxyethyl ene castor oil | - | - | - | 0.05 | 0.1 | - | - |
| Polysorbate 80 | 0.3 | 0.2 | - | - | - | 0.2 | 0.2 |
| Polyoxyl stearate | - | - | - | - | 0.1 | - | - |
| Polyoxyethyl ene polypropylen e glycol | - | - | - | - | 0.05 | - | - |
| Benzalkonium chloride | - | - | - | - | - | - | 0.0001 |
| Chlorhexidine gluconate | - | - | - | - | - | - | 0.0001 |
| Polyhexanide hydrochloride | - | - | - | - | - | - | 0.0001 |
| Chlorobutanol | 0.15 | - | - | - | - | - | - |
| Sodium edetate | - | 0.001 | - | - | - | - | 0.01 |
| Boric acid | 0.5 | 1 | 0.4 | 1 | - | 1 | 1 |
| Borax | - | 0.1 | 0.1 | 0.2 | - | 0.2 | 0.2 |
| Sodium hydrogen phosphate | - | - | - | - | 0.15 | - | - |
| Sodium dihydrogen phosphate | - | - | - | - | 0.1 | - | - |
| Sodium citrate | - | - | 0.001 | - | - | 0.01 | 0.1 |
| Sesame oil | - | 0.001 | - | - | 0.05 | - | - |
| Propylene glycol | - | - | - | - | 0.1 | - | - |
| Fluidized paraffin | - | - | - | 0.05 | - | - | - |
| Concentrated glycerin | - | - | - | - | - | 0.1 | - |
| L-alginine | - | 0.5 | - | - | - | - | - |
| Hydrochloric acid | Appropriate amount | Appropriate amount | Appropriate amount | Appropriate amount | Appropriate amount | Appropriate amount | Appropriate amount |
| Sodium hydroxide | Appropriate amount | Appropriate amount | Appropriate amount | Appropriate amount | Appropriate amount | Appropriate amount | Appropriate amount |
| Purified water | Residue | Residue | Residue | Residue | Residue | Residue | Residue |
| Total amount (mL) | 100 mL | 100 mL | 100 mL | 100 mL | 100 mL | 100 mL | 100 mL |
| pH | 7 | 6.8 | 6.8 | 6.3 | 7.5 | 7 | 6.8 |

## Claims

1. An ophthalmological composition comprising:
zinc chloride (A); and
a polyvinyl-based polymer compound (B).

2. The ophthalmological composition according to Claim 1,
wherein the polyvinyl-based polymer compound (B) is polyvinyl pyrrolidone.

3. The ophthalmological composition according to Claim 2,
wherein a content of the polyvinyl pyrrolidone is 0.001 to 5 w/v% based on a total amount of the ophthalmological composition.

4. The ophthalmological composition according to Claim 2 or 3,
wherein the polyvinyl pyrrolidone has a viscosity characteristic value of 10 to 150.

5. The ophthalmological composition according to any one of Claims 1 to 4 that is for suppressing eye dryness or dry eye.
